Europäisches Patentamt

European Patent Office

Office européen des brevets

(19)

(11) Veröffentlichungsnummer: **0 303 056**
**A1**

## (12) EUROPÄISCHE PATENTANMELDUNG

(21) Anmeldenummer: 88111012.6

(22) Anmeldetag: 09.07.88

(51) Int. Cl.4: **A61B 5/02 , A62B 18/08 , A61M 16/00**

(30) Priorität: 23.07.87 DE 3724336

(43) Veröffentlichungstag der Anmeldung:
**15.02.89 Patentblatt 89/07**

(84) Benannte Vertragsstaaten:
**DE FR GB SE**

(71) Anmelder: **Drägerwerk Aktiengesellschaft**
**Moislinger Allee 53-55**
**D-2400 Lübeck 1(DE)**

(72) Erfinder: **Töpfer, Lothar, Dipl.-Ing.**
**Langenbroich 38**
**D-4030 Ratingen 6(DE)**
Erfinder: **Weiland, Christine, Dr. med.**
**Königshügel 27**
**D-5100 Aachen(DE)**
Erfinder: **Rath, Alfred, Dipl.-Ing.**
**Sandberg 16**
**D-2401 Hamberge(DE)**

(54) **Schutzmaske mit eingebautem Sensor zur Überwachung von Lebensfunktionen.**

(57) Eine Vorrichtung zur Überwachung von Lebensfunktionen, mit mindestens einem am Körper gehaltenen Sensor, soll derart verbessert werden, daß, insbesondere bei Verwendung schweren Atemschutzes, ein einfaches und zuverlässiges Anlegen und auch unter erschwerten Einsatzbedingungen sicheres Anliegen des Sensors ermöglicht wird. Zur Befestigung des Sensors sollen keine zusätzlichen, ausschließlich für den Zweck seiner Halterung vorgesehenen und evtl. störenden Hilfsmittel notwendig sein. Dazu ist der Sensor innenseitig einer Atemschutzmaske angeordnet.

EP 0 303 056 A1

## Schutzmaske mit eingebautem Sensor zur Überwachung von Lebensfunktionen

Die Erfindung betrifft eine Vorrichtung zur Überwachung von Lebensfunktionen, mit mindestens einem am Körper gehalterten Sensor.

Eine derartige Vorrichtung ist aus der DE-OS 31 06 315 bekanntgeworden.

Die bekannte Vorrichtung dient zur Überwachung des Blutkreislaufes, Messung des Blutdruckes, zur Selbstüberwachung von Kreislaufkranken, aber auch von Menschen, die beispielsweise im Berufsleben oder im Sport unter besonderer ärztlicher Überwachung stehen müssen.

Der Bedarf an Überwachung von Lebensfunktionen ist jedoch nicht nur auf den medizinischen Sektor beschränkt, sondern ist auch für Personen, die beispielsweise unter schwerem Atemschutz Rettungsarbeiten verrichten müssen, wie z. B. Feuerwehrleute im Einsatz oder in Übungsstrecken, sowie Rettungs- und Bergungstaucher oder Mitarbeiter des Grubenrettungswesens gegeben.

Derartige Betätigungen unter erschwerten Bedingungen bedeuten eine extreme Belastung für die Leistungsfähigkeit des menschlichen Körpers, die leicht überschätzt wird, wenn insbesondere unter Zeitdruck Menschenleben gerettet werden müssen. Es besteht dann häufig die Gefahr, daß während der Rettungs- bzw. Bergungsarbeiten die Grenze der Belastbarkeit zu spät erkannt und der Rettungseinsatz dadurch gefährdet wird, daß die betreffende Rettungsperson für den Einsatz ausfällt, ohne daß es überwachbar ist und bemerkt werden kann.

Die bekannte Vorrichtung sieht vor, daß zur Überwachung von Lebensfunktionen ein entsprechender Sensor an dem Bügel eines Brillengestelles befestigt ist und durch den Spanndruck, evtl. unterstützt durch ein die Brillenbügel zusammenziehendes Band, gegen den Schläfenbereich des Trägers gedrückt ist.

Nachteilig bei der bekannten Anordnung ist es, daß in jedem Falle ein Brillengestell getragen werden muß, auch wenn die betreffende Person kein Brillenträger ist. Hinzu kommt, daß bei Tragen einer Gesichtsmaske ein derartiges Brillengestell unnötigerweise die freie Beweglichkeit behindert; beim Tragen von Vollmasken ist die erforderliche Abdichtung nicht gewährleistet.

Außerdem kann durch Schweißbildung auf der Haut des Trägers während der angestrengten Arbeitstätigkeit der Kontakt zwischen Hautoberfläche und Sensor durch Verrutschen gelöst werden.

Der vorliegenden Erfindung liegt somit die Aufgabe zugrunde, eine Vorrichtung zur Überwachung von Lebensfunktionen derart zu verbessern, daß, insbesondere bei Verwendung eines schweren Atemschutzes, ein einfaches und zuverlässiges Anlegen und auch unter erschwerten Einsatzbedingungen sicheres Anliegen des Sensors ermöglicht wird. Zur Befestigung des Sensors sollen keine zusätzlichen, ausschließlich für den Zweck der Sensorhalterung vorgesehenen und evtl. störenden Hilfsmittel notwendig sein.

Die Lösung der Aufgabe erfolgt dadurch, daß der Sensor innenseitig einer Atemschutzmaske angeordnet ist.

Die Vorteile der Erfindung sind im wesentlichen darin zu sehen, daß nunmehr schon bei Anlegen der Atemschutzmaske, vorzugsweise einer Vollmaske, automatisch der entsprechende Sensor an der für ihn geeigneten Stelle, erforderlichenfalls unter Druck, dem Gesichtsbereich des Trägers anliegt. Je nach gewünschter Überwachung verschiedener Funktionen können mehrere Sensoren vorgesehen sein, die, je nach Ausführungsform und Aufgabe, der Gesichtsoberfläche des Maskenträgers anliegen oder auch im Innenraum der Atemschutzmaske angebracht sind. Solche Sensoren können beispielsweise der Messung von Herzfrequenz, Atemfrequenz, Atemdrücken oder Körpertemperaturen dienen.

Ein besonders günstiger Anbringungsort für den Sensor bezüglich einer unverrutschbaren und auch platzsparenden Befestigung bietet sich in dem Dichtrahmen der Atemschutzmaske an. Um die Dichtfunktion der Vollmaske zu gewährleisten, ist ein festes Anliegen des Dichtwulstes am Gesichtsumfang notwendig, und die dafür vorgesehenen vorhandenen Hilfsmittel, wie z. B. Maskenbänderung, erlauben es, die Vollmaske auch unter erschwerten Arbeitsbedingungen fest anliegen zu lassen. Dadurch wird gleichzeitig ein festes und unverrutschbares Anliegen des Sensors auf der Hautoberfläche sichergestellt.

Ein besonders vorteilhafter Anbringungsort für den Sensor bietet sich in der Höhlung einer Doppelwulstdichtung an. Dadurch ergibt sich ein erhöhter Schutz des Sensors vor äußeren Einflüssen.

Zweckmäßigerweise werden als Sensor ein Herzfrequenzabnehmer oder ein Kontaktthermometer zur Bestimmung der Körpertemperatur vorgesehen.

Ein Ausführungsbeispiel der Erfindung wird anhand einer schematischen Darstellung gezeigt und im folgenden näher erläutert.

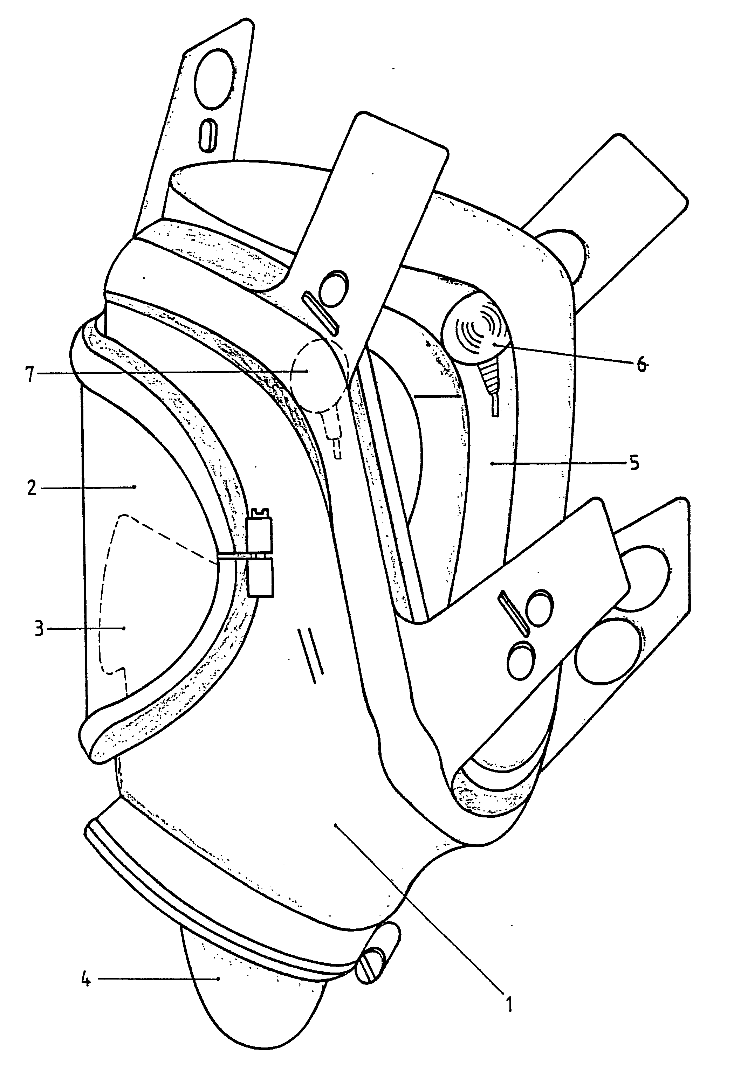

Die einzige Figur zeigt eine Vollmaske in der Ansicht der dem Maskenträger zugewandten Seite. Der Maskenkörper (1) enthält eine Sichtscheibe (2) und eine Innenhalbmaske (3), welche weiterhin ein Ausatemventil (4) trägt. Der Maskenkörper (1) ist ringsum von einem Dichtrahmen (5) umgeben. der im Schläfenbereich des Maskenträgers einen Sensor (6) zur Abnahme der Herzfrequenz aufnimmt. Dieser Sensor (6) kann beispielsweise als ein druckempfindliches Element ausgebildet sein, welches den Pulsdruck der Schläfenarterie aufspürt und ein entsprechendes Signal entweder über eine nicht dargestellte Meßleitung oder auch telemetrisch an ein ebenfalls nicht dargestelltes Auswertegerät zur Anzeige der Herzfrequenz abgibt.

Ein zusätzliches Kontaktthermometer (7) kann an der dem Sensor (6) gegenüberliegenden Seite des Dichtrahmens (5) angeordnet sein, um die Körpertemperatur abzugreifen.

## Ansprüche

1. Vorrichtung zur Überwachung von Lebensfunktionen, mit mindestens einem am Körper gehalterten Sensor, dadurch gekennzeichnet, daß der Sensor (6) innenseitig einer Atemschutzmaske (1) angeordnet ist.

2. Vorrichtung nach Anspruch 1, dadurch gekennzeichnet, daß als Halterung für den Sensor (6) der Dichtrahmen (5) der Atemschutzmaske (1) vorgesehen ist.

3. Vorrichtung nach Anspruch 2, dadurch gekennzeichnet, daß der Sensor (6) in der Höhlung einer Doppelwulstdichtung (5) angeordnet ist.

4. Vorrichtung nach einem der Ansprüche 1 bis 3, dadurch gekennzeichnet, daß als Sensor ein Temperaturaufnehmer (7) und/oder ein Herzfrequenzabnehmer (6) vorgesehen sind.

Europäisches Patentamt

**EUROPÄISCHER RECHERCHENBERICHT**

Nummer der Anmeldung

EP 88 11 1012

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | CA-A-1 123 705 (H. ROLAND) * Seite 3, Zeile 11 – Seite 4, Zeile 15; Seite 7, Zeile 22 – Seite 9, Zeile 6; Figuren 2,3 * | 1 | A 61 B 5/02 A 62 B 18/08 A 61 M 16/00 |
| X | GB-A-2 130 893 (NATIONAL RESEARCH DEVELOPMENT CORP.) * Zusammenfassung; Figuren 1,2 * | 1 | |
| A | FR-A-2 116 832 (R. DELEST) * Seite 1, Zeilen 1-25; Figuren 1-7 * | 1 | |
| A | DD-C- 253 943 (P. KRAUSE et al.) * Seite 2, Zeilen 1-35; Figur 1 * | 1 | |
| A | CH-A- 621 056 (W. MUELLER) * Zusammenfassung; Figur 1 * | 4 | |
| A | DE-B-1 234 532 (DRAEGERWERK HEINR. & BERNH. DRAEGER) * Spalte 3, Zeilen 1-7 * | 3 | |

**RECHERCHIERTE SACHGEBIETE (Int. Cl.4)**

A 62 B 18/00
A 61 M 16/00
A 61 B 5/00

Der vorliegende Recherchenbericht wurde für alle Patentansprüche erstellt

| Recherchenort | Abschlußdatum der Recherche | Prüfer |
|---|---|---|
| BERLIN | 11-11-1988 | WEIHS J.A. |